(19)

Europäisches
Patentamt
European
Patent Office
Office européen
des brevets

(11)  **EP 1 803 393 A1**

(12)  **EUROPEAN PATENT APPLICATION**
published in accordance with Art. 158(3) EPC

(43) Date of publication:
**04.07.2007  Bulletin 2007/27**

(51) Int Cl.:
**A61B 5/055** (2006.01)

(21) Application number: **05793156.0**

(22) Date of filing: **12.10.2005**

(86) International application number:
**PCT/JP2005/018781**

(87) International publication number:
**WO 2006/041084 (20.04.2006 Gazette 2006/16)**

(84) Designated Contracting States:
**DE FR GB IT NL**

(30) Priority: **13.10.2004  JP 2004298304**

(71) Applicant: **HITACHI MEDICAL CORPORATION**
**Tokyo (JP)**

(72) Inventors:
• **TAKIZAWA, Masahiro**
**2770813 (JP)**

• **TAKAHASHI, Tetsuhiko**
**1010021 (JP)**

(74) Representative: **Buchetmann, Dominik**
**Strehl Schübel-Hopf & Partner**
**Maximilianstrasse 54**
**80538 München (DE)**

(54)  **MAGNETIC RESONANCE IMAGING DEVICE AND METHOD**

(57)    A magnetic resonance imaging apparatus including signal receiving means for detecting a nuclear magnetic resonance signal from an object, signal processing means for reconstructing an image by using the detected nuclear magnetic resonance signal and display means for displaying the image, a whole image of the examiner being obtained while each imaging site of the object is continuously or stepwise moved and disposed in the imaging space, is equipped with detecting means for detecting the gradient and size of each site of the object, inputting means for inputting reference information for carrying out magnetic resonance imaging corresponding to the gradient and size of each site of the object onto an image representing the gradient and size of each site of the object which is displayed on the display means, storage means for storing the input reference information, control means for controlling the imaging operation on the basis of the reference information stored in the storage means, and combining means for combining nuclear magnetic resonance signals obtained through the imaging operation carried out under the control to create the whole image.

## FIG. 6

**Description**

Technical Field

The present invention relates to magnetic resonance imaging (hereinafter MRI) apparatus and method, and particularly to MRI device and method that can perform imaging in accordance with the situation that the arrangement direction or the size is different among respective sites of an object in MRI for imaging a broad range or the whole body of the obj ect.

Background Art

In the MRI apparatus, a nuclear magnetic resonance signal (hereinafter referred to as NMR signal) from an object is detected by using a nuclear magnetic resonance (hereinafter referred to as NMR) phenomenon occurring in atomic nucleuses of atoms constituting the object when electromagnetic waves are irradiated to the object placed in a uniform magnetostatic field, and images are re-constructed by using this NMR signal, thereby achieving magnetic resonance images (hereinafter referred to as MR image) representing the physical properties of the object.

In the field of MRI, there is known a technique in which an object is placed on a table, a broad range or the whole body of the object is imaged while the table is moved continuously or stepwise in the gantry of an MRI apparatus. In such a technique, for example, an imaging slice section is set in parallel to the moving direction of the table, and then the broad range or the whole body of the obj ect is imaged while moving the table (see a non-patent document 1 as an example when the table is continuously moved, a patent document 1 as an example when the table is moved stepwise, for example).

Non-patent document 1: Kruger DG, Riederer SJ, Grimm RC, Rossman PJ.: Continuously Moving Table Data Acquisition Method for Long FOV Contrast-Enhanced MRA and Whole-Body MRI. Magnetic Resonance in Meddicine 47(2):224-231 (2002)

Patent Document 1: USP no. 6,311,085

However, as an inspection result of the above prior art, the inventors of this applicant have found the following problem.

That is, the imaging slice section which is set in parallel to the table plane or the moving direction of the table in the prior art has a thickness which is the same level as or less than the thickness of the body of an object who normally is laid down on his/her back. However, in such a case that the object is laid down on the table while his/her knee or the like is bent, the bent knee may protrude out of the imaging slice section set as described above. That is, when a part of the object has a gradient or has a different size from the other sites, there is a problem that a part of the subj ect matter protrudes out of the imaging slice section. That is, the prior art pays no attention to the arrangement situation of each site of the subject matter (a case where the object is put in an oblique position or the like).

Disclosure of the Invention

An object of the present invention is to provide MRI apparatus and method that can perform an imaging operation in accordance with the situation that the arrangement direction or size is different among respective sites of an object in MRI for imaging a broad range or the whole body of the object.

In order to attain the above object, an MRI apparatus of the present invention which is equipped with static magnetic field generating means for generating static magnetic field in an imaging space, gradient magnetic field generating means for generating gradient magnetic field in the imaging space, radio frequency magnetic field generating means for generating radio frequency magnetic field so as to induce nuclear magnetic resonance to an obj ect disposed in the imaging space, signal receiving means for detecting a nuclear magnetic resonance signal from the object, signal processing means for reconstructing an image by using the detected nuclear magnetic resonance signal, display means for displaying the image, a table for disposing the object in the imaging space while the object is put on the table, and table moving means for moving the table on which the object is put and in which the overall image of the object is obtained while each imaging site of the object is continuously or stepwise moved in the imaging space, thereby performing magnetic resonance imaging, is characterized by further comprising:

detecting means for detecting the gradient and size of each site of the object, the gradient and size of each site of the object that is detected by the detecting means being displayed on the display means;

input means for inputting reference information for carrying out magnetic resonance imaging in conformity with the gradient and the size onto an image representing the gradient and size of each site of the object that is displayed on the display means;

storage means for storing the input reference information;

control means for controlling the imaging operation on the basis of the reference information stored in the storage means; and

combining means for combining nuclear magnetic resonance signals obtained through the imaging operation executed under the control to generate the overall image.

An MRI method in magnetic resonance imaging for imaging a broad range or the whole body of an object while a table on which the object is laid down is moved, is characterized by comprising:

(1) a step of inputting reference information in accordance with an arrangement situation of each site of the object;
(2) a step of performing imaging by using the reference information; and
(3) a step of synthesizing an overall image by using nuclear magnetic resonance signals obtained through the step (2).

Brief Description of the Drawings

[Fig. 1] is a diagram showing a general MRI apparatus according to the present invention.
[Fig. 2] (a) is a diagram showing an example of an imaging pulse sequence, (b) is a diagram showing an example in which an echo signal is disposed on k space.
[Fig. 3] (a) is a diagram showing how the relationship between an object and an imaging space varies as the table is moved, (b) is a diagram showing a method of performing imaging while the table is continuously moved.
[Fig. 4] (a) is a view taken when an object who is laid down on the table is viewed from the upper side in the vertical direction, (b) is a view taken when the object who is laid down on the table is viewed from just the side thereof, (c) is a diagram showing hybrid data obtained by subjecting an echo signal to one-dimensional Fourier Transform in a reading gradient magnetic field direction (ky direction), and (d) is a diagram showing an example in which hybrid data having corresponding phase encode amount are connected.
[Fig. 5] is a flowchart showing the process of MRI of an embodiment 1.
[Fig. 6] is a diagram showing a set example of imaging block.
[Fig. 7] (a) is a diagram showing a set example of an imaging slice section into an imaging block, (b) is a diagram showing how the imaging slice section is switched when moving to an imaging block having a different gradient, (c) is a diagram showing an overall image generated in the embodiment 1.

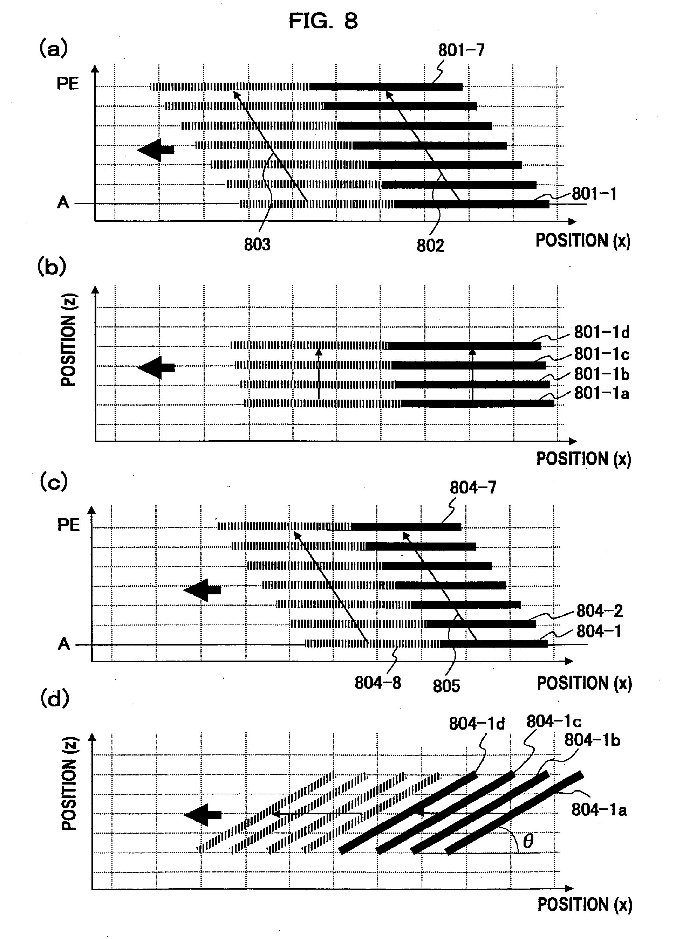

[Fig. 8] (a) is a diagram showing an aspect that an imaging operation is carried out while the imaging slice section is switched when the imaging block is parallel to the moving direction of the table, and viewed from an x-ky plane, (b) is a diagram showing an aspect that the imaging operation is carried out while the imaging slice section is switched when the imaging block is parallel to the moving direction of the table, and viewed from an x-z plane, (c) is a diagram showing an aspect that the imaging operation is carried out while the imaging slice section is switched when the imaging block is inclined with respect to the moving direction of the table, and viewed from an x-ky plane, and (d) is a diagram showing an aspect that the imaging operation is carried out while the imaging slice section is switched when the imaging block is inclined with respect to the moving direction of the table, and viewed from an x-z lane.

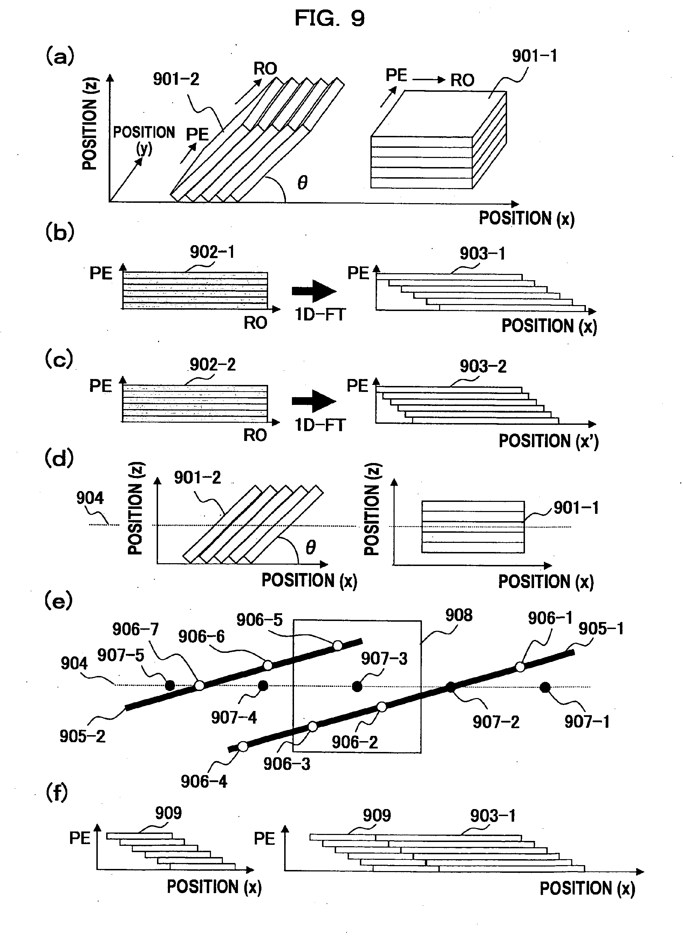

[Fig. 9] (a) is a diagram showing an example in which the imaging slice section is indicated while varying the angle every imaging block, (b) is a diagram (left side) showing a direct arrangement of echo signal data measured in slab 901-1 and a diagram (right side) showing that 902-1 is subjected to Fourier Transform in the direction of the application of the reading gradient magnetic field and further arranged as hybrid data by making the positions thereof on the x-axis proper (right side), (c) is a diagram (left side) showing a direct arrangement of echo signal data measured in slab 902-2 and a diagram (right side) showing that 902-2 is subjected to Fourier Transform in the direction of the application of the reading gradient magnetic field and further arranged as hybrid data by making the positions thereof on the x-axis proper (right side), (d) is a diagram showing that the hybrid data obtained in Figs. 9 (b) and (c) are added with position information in the z-axis direction and arranged on a virtual three-dimensional space (the left side is a diagram corresponding to the slab 901-2, and the right side is a diagram corresponding to the slab 90-1), (e) is a diagram showing the interpolation processing of the hybrid data, and (f) is a diagram showing an aspect of connecting hybrid data by different slabs.

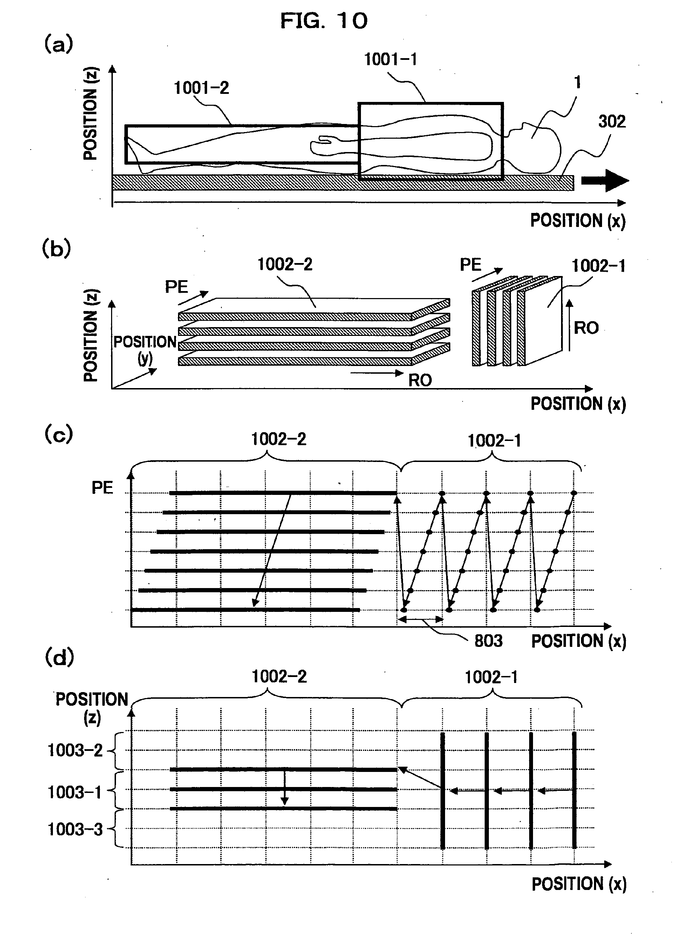

[Fig. 10] (a) is a diagram showing a set example of an imaging block according to an embodiment 3, (b) is a diagram showing an imaging slice section of the embodiment 3 and a set example of the directions of the reading gradient magnetic field and the phase encode gradient magnetic field, (c) is a cross-sectional view obtained when hybrid data arranged on the virtual three-dimensional hybrid space of the embodiment 3 are cut out by a section parallel to the x-ky plane, and (d) is a cross-sectional view obtained when the hybrid data arranged on the virtual three-dimensional hybrid space of the embodiment 3 are cut out by a section parallel to the x-z plane.

[Fig. 11] is a flowchart of an embodiment 4.
[Fig. 12] is a diagram showing a display example of a positioning image in the embodiment 4.

Best Modes for carrying out the Invention

Embodiments according to the present invention will be described hereunder with reference to the drawings. In all the figures for describing the embodiments of the present invention, the elements having the same functions are represented by the same reference numerals, and the repetitive description thereof is omitted.

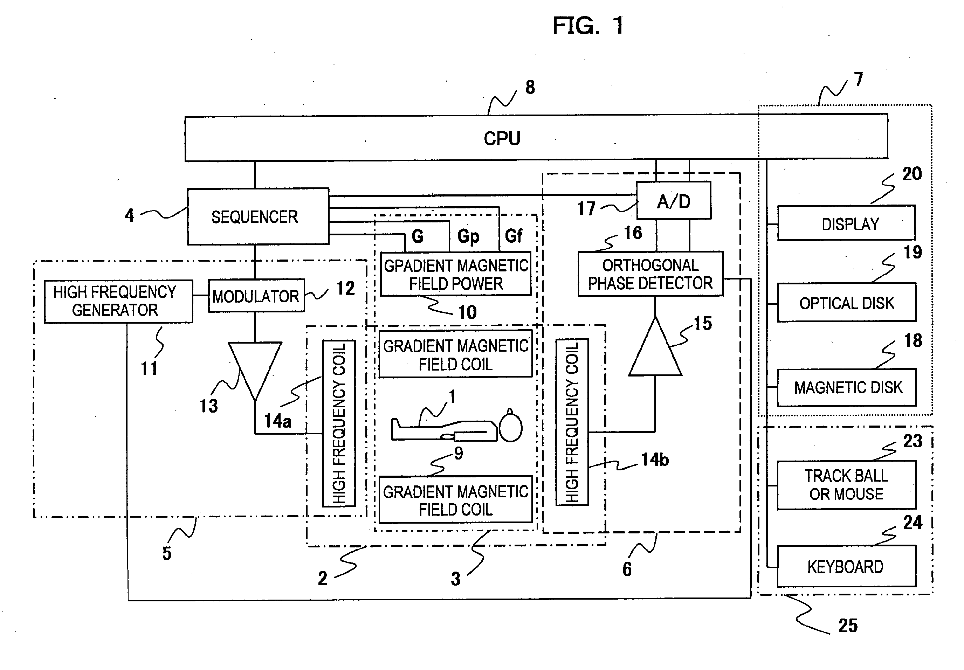

First, a general MRI apparatus according to the present invention will be described with reference to Fig. 1. Fig. 1 is a block diagram showing the overall construction of an MRI apparatus according to the present invention. The magnetic resonance imaging apparatus obtains tomograms of an obj ect by using the nuclear magnetic resonance (NMR) phenomenon, and it is constructed by a static magnetic field generating system 2, a gradient magnetic field generating system 3, a transmission system 5, a reception system 6, a signal processing system 7, a sequencer 4, and a central processing unit (CPU) 8 as shown in Fig. 1.

The static magnetic field generating system 2 generates uniform static magnetic field in the body axis of an object 1 or in a direction perpendicular to the body axis in a space surrounding the obj ect 1, and permanent magnet type, a normal conduction type or superconduction type of magnetic field generating means is disposed around the object 1.

The gradient magnetic field generating system 3 comprises a gradient magnetic field coils 9 which are wound in the three axial direction of X, Y, Z, and a gradient magnetic field power source 10 for driving the respective gradient magnetic field coils. By driving the gradient magnetic field power source 10 for the respective coils according to an instruction from a sequencer 4 described later, gradient magnetic field Gs, Gp, Gf in the three axial direction of X, Y, Z are applied to the obj ect 1. More specifically, a gradient magnetic field pulse (Gs) in a slice direction is applied to one of the X, Y and Z directions to set a slice plane for the object 1, and a gradient magnetic field pulse (Gp) in a phase encode direction and a gradient magnetic field pulse (Gf) in a frequency encode direction are applied to the remaining two directions, whereby an echo signal is encoded with position information of the respective directions. Or, oblique gradient magnetic field is applied by using a technique as disclosed in JP-A-7-23931 to perform an oblique imaging operation.

The sequencer 4 is control means for repetitively applying a radio frequency magnetic filed pulse (hereinafter "RF pulse") and a gradient magnetic field pulse at a predetermined pulse sequence. It is operated under the control of CPU 8, and transmits various instructions necessary to collect the data of tomograms of the object 1 to the transmission system 5, the gradient magnetic field generating system 3 and the reception system 6.

The transmission system 5 irradiates the RF pulse for inducting nuclear magnetic resonance in atomic nuclei spins of atoms constituting an anatomy of the object 1, and it comprises a high frequency oscillator 11, a modulator 12, a high frequency amplifier 13 and a high frequency coil 14a at the transmission side. The high frequency pulse output from the high frequency oscillator 11 is subjected to amplitude modulation by the modulator 12 at the timing instructed from the sequencer 4. The amplitude-modulated high frequency pulse is amplified by the high frequency amplifier 13, and supplies to the high frequency coil 14a disposed in proximity to the object 1, whereby the electromagnetic wave (RF pulse) is irradiated to the object 1.

The reception system 6 detects an echo signal (NMR signal) discharged through the nuclear magnetic resonance of atomic nuclei spins of atoms constituting an anatomy of the object 1, and it comprises a high frequency coil 14b at the reception side, an amplifier 15, an orthogonal phase detector 16 and an A/D converter 17. A responding electromagnetic wave (NMR signal) of the object 1 which is induced by the -electromagnetic wave irradiated from the high frequency coil 14a at the transmission side is detected by the high frequency coil 14b disposed in proximity to the object 1, and amplified by the amplifier 15. Thereafter, the electromagnetic wave is divided into orthogonal signals of two system at the timing based on the instruction from the sequencer 4 by the orthogonal phase detector 16. Each of these signals is converted to a digital quantity in an A/D converter 17, and then transmitted to a signal processing system 7.

The signal processing system 7 has an external storage device such as a magnetic disc 18, an optical disc 19, or the like, and a display 20 comprising CRT, etc. When data are input from the reception system 6 into CPU 8, CPU 8 executes signal processing, processing of reconstructing images, etc., and it displays the tomogram of the object 1 as a processing result on the display 20 and records the processing result into a magnetic disc 18 of the external storage device or the like.

An operating portion 25 inputs various kinds of control information of the MRI apparatus and control information for the processing executed in the signal processing system 7, and it comprises a track ball, a mouse 23 and a keyboard 24. The operating portion 25 is disposed in proximity to the display 20, and an operator interactively controls various kinds of processing of the MRI apparatus through the operating portion 25 while watching the display 20.

In Fig. 1, the high frequency coils 14a and 14b and the gradient magnetic field coils 9 at the transmission side and the reception side are disposed in the static magnetic field space of the static magnetic field generating system 2 disposed in the space surrounding the object 1.

Proton, a main constituent material of an object, is known as a present imaging target spin species which has been popular in clinical medicine. By imaging the spatial distribution of proton density or the spatial distribution of a relaxation phenomenon of an excitation state, the conformation or function of the head, an abdominal part, four limbs, etc. of a human body is two-dimensionally or three-dimensionally displayed.

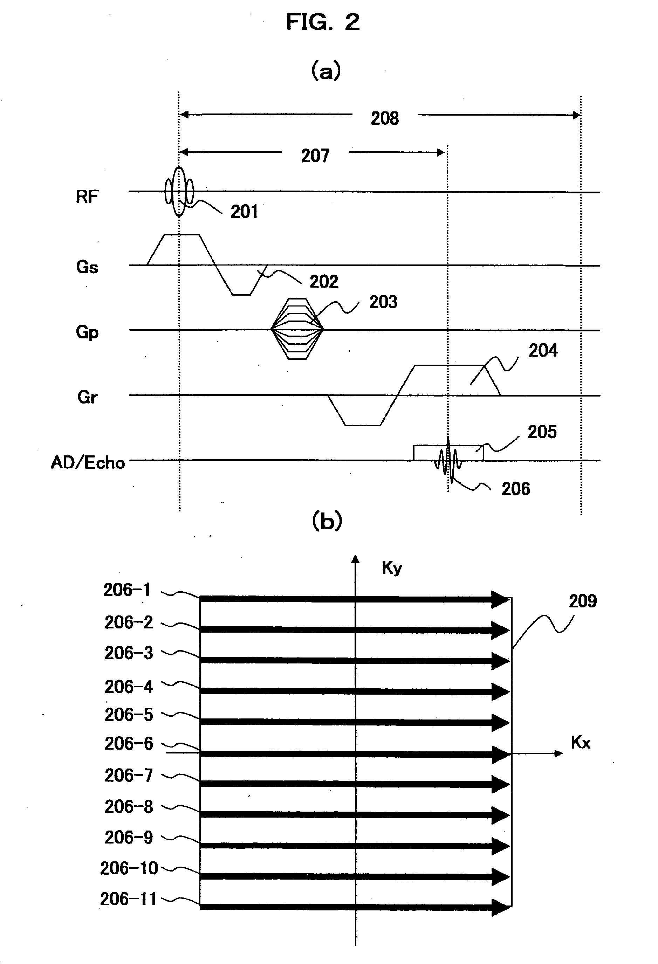

Next, an example of an imaging pulse sequence of the present invention will be described. Fig. 2(a) shows a gradient echo pulse sequence. In Fig. 2 (a), RF, Gs, Gp, Gr, AD/echo are axes representing application of an RF pulse, a slice selection gradient magnetic field, phase encode gradient magnetic field, and reading gradient magnetic field, and execution of AD conversion/measurement of echo signal, 201 represents the RF pulse, 202 represents a slice selection gradient magnetic field pulse, 203 represents a phase encode gradient magnetic field pulse, 204 represents a reading gradient magnetic field pulse, 205 represents a sampling window for executing AD conversion, and 206 represents an echo signal to be measured.

The measurement of the echo signal is repetitively executed at a time interval 208 (repetitive time TR), and each echo signal occurs after a time 207 (echo time TE) from application of the RF pulse 201. The obtained echo signal 206 is disposed in a k space 209 shown in Fig. 2(b). The abscissa axis kx of Fig. 2(b) corresponds to the time of the sampling window 205 of the echo signal in Fig. 2(a), and the ordinate axis ky corresponds to the phase encode amount (the area of a wave shape) applied by the phase encode gradient magnetic field pulse 203 in the Gp axis of Fig. 2(a).

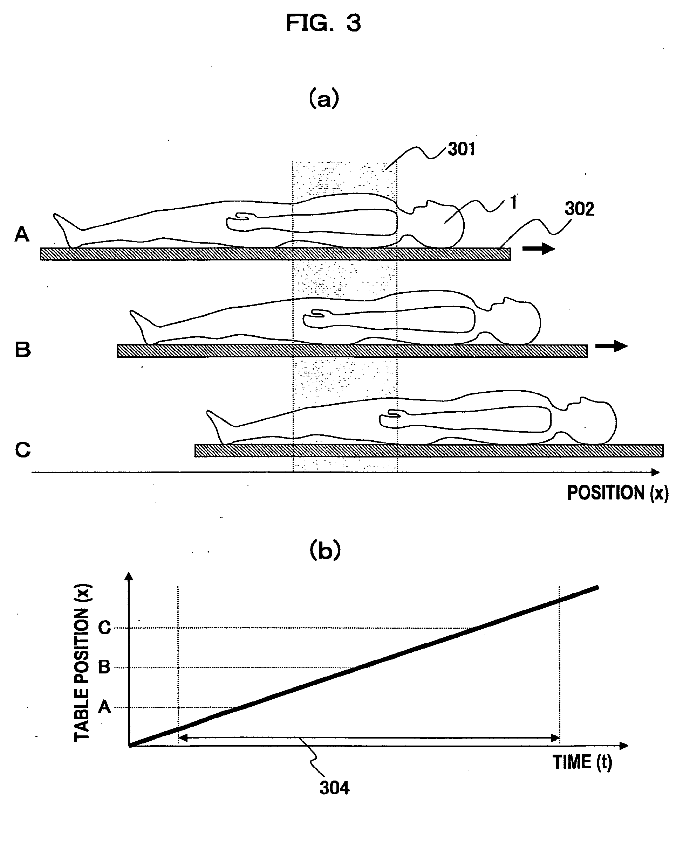

Next, the conceptual diagram of MRI for imaging a broad range or the whole body of an object while continuously moving a table in the gantry of the MRI apparatus will be described with reference to Fig. 3.

First, Fig. 3(a) is a diagram showing how the relationship between the object and the imaging space varies in accordance with the movement of the table. In Fig. 3(a), 301 represents the imaging space, and 302 represents the table. The object 1 is put on the table 302, and the table 302 is freely moved in the x-axis direction by table moving means (not shown). The positional relationship between the imaging space 301 and the object 1 is varied in accordance with the movement of the table 302, whereby images of different sites of the object can be obtained. For example, in the case of a positional relationship indicated by A, the breast portion of the object is imaged, and in the case of a positional relationship indicated by B or C, the abdominal part and the leg portion are imaged, respectively.

Fig. 3(b) shows a method of performing imaging while continuously moving the table. According to this method, the moving speed of the table is normally fixed over the whole period 304 of the position A, the position B, the position C, etc.

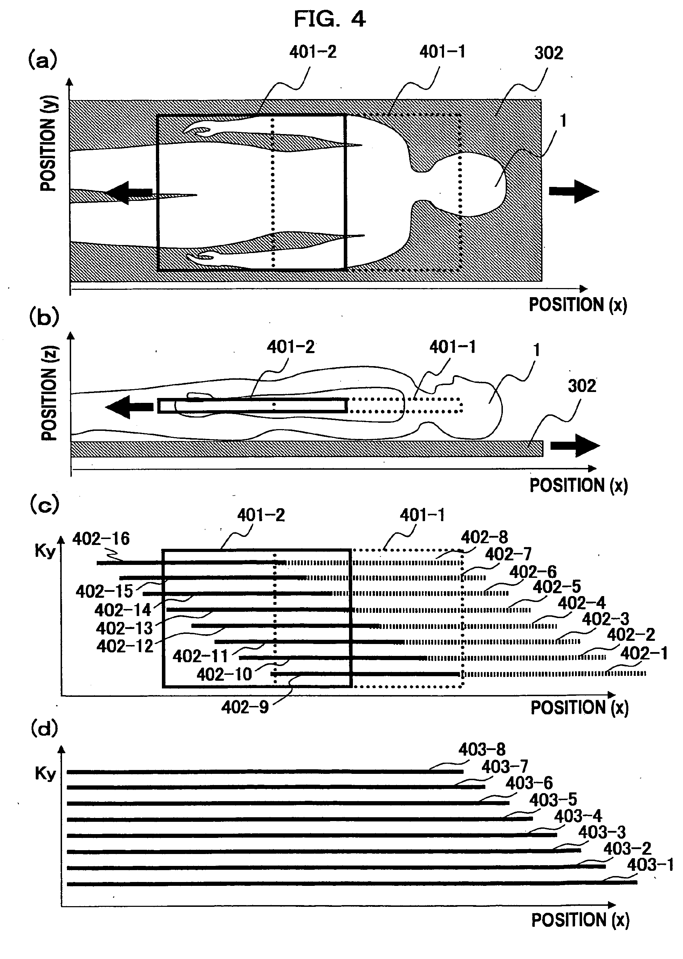

The image reconstruction of this method is carried out by using the echo signal obtained at each table position which is continuously moved. Next, the details (the image reconstructing method, etc.) of the imaging method executed while the table is continuously moved as shown in Fig. 3(b) will be described with reference to Fig. 4. Fig. 4 shows an example in which an imaging slice section is set in parallel to the moving direction of the table 302. The description will be made in turn with reference to Figs. 4(a) to (d).

First, Fig. 4(a) shows an object laid down on the table 302 which is viewed from the upper side in the vertical direction, and Fig. 4(b) is a view taken from the side. In Figs. 4 (a) and (b), 401-1 and 401-2 shows that the imaging view field is moved from 401-1 to 401-2. More specifically, when the table 302 moves to the plus side in the x direction, the imaging view field moves from 401-1 to 401-2, and the imaging view field moves to the minus side in the x-direction with respect to the object.

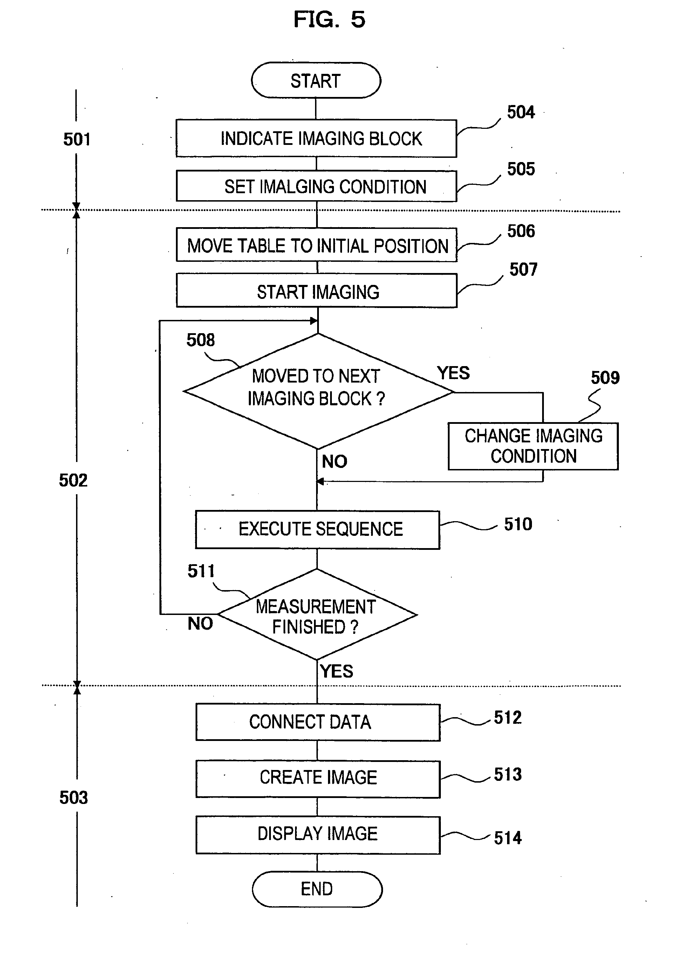

In this imaging method, the direction of the reading gradient magnetic field pulse applied when each echo is collected is parallel to the moving direction of the table, and the intensity thereof is fixed. On the other hand, with respect to the phase encode gradient magnetic field pulse applied to collect each echo, the direction thereof is a horizontal direction (the y direction shown in Fig. 4 (a)) which is orthogonal to the moving direction of the table, and the phase encode amount applied when each echo is collected is recursively varied. When the obtained echo signal is subjected to one-dimensional Fourier Transform in the direction (kx direction) of the reading gradient magnetic field, hybrid data (data obtained by subjecting k space to Fourier Transform in only one direction) shown by the Fig. 5(c) are obtained. In the hybrid data in Fig. 5(c), the abscissa axis corresponds to the x axis representing the position in the table moving direction, and they are arranged at the respective positions.

Furthermore, the ordinate axis represents the phase encode amount in the horizontal direction perpendicular to the moving direction of the table, and the data obtained by subjecting the respective echo signals to one-dimensional Fourier Transform in the reading direction (kx direction) are arranged at the respective positions.

Since the phase encode amount is recursively varied, the respective data 402-1 to 402-8 are associated and connected to the respective data 402-9 to 402-16. Therefore, in Fig. 4(c) by connecting 402-9 to 402-1, connecting 402-10 to 402-2, etc. in association with each other, the data 403-1 to 403-8 shown in Fig. 5(d) are obtained. In Fig. 4 (d), 403-1 to 403-8 are data different in phase encode amount, and by subjecting the data to Fourier Transform in the ky direction, a final image is obtained (see Non-patent Document 1 for the detailed explanation).

In the imaging based on the continuous movement of the table as described above, it is necessary for connecting the hybrid data without discontinuing the imaging area that the moving speed of the table is controlled in connection with the execution of the pulse sequence (for example, parameters such as repetitive time, etc.) or conversely the execution of the pulse sequence (for example, parameters such as repetitive time, etc.) is controlled corresponding to the table moving speed.

Furthermore, in the imaging based on the continuous movement of the table as described above, a broad range in the table moving direction of the object can be obtained by only one image. Furthermore, there is no step of joining images picked up every step as in the case of the method of moving the table stepwise, and thus there is an advantage that no positional displacement occurs in the joint step.

Embodiment 1

An embodiment 1 of the present invention will be described with reference to the flowchart of Fig. 5 and Figs. 6 to 8, Fig. 9(a). In this embodiment, when the continuous imaging based on the table movement is carried out as shown in Figs. 3 (b) and Fig. 4, the imaging section is divided into plural imaging blocks corresponding to the arrangement condition of each site of the object, and the setting of the gradient of the imaging slice section, etc. is changed every imaging block to obtain data. Plural data obtained on the basis of different imaging slice sections are connected to one another to obtain an image of a broad range or the whole body. In this embodiment, the gradient echo pulse sequence shown in Fig. 2 is used as an imaging sequence. First, the description will be made in turn with reference to the flowchart of Fig. 5.

According to the flowchart of Fig. 5, the imaging process of this embodiment comprises a prearrange step group 501 for indicating imaging blocks, etc. as a pre-stage of this imaging operation, a step group 502 for carrying out this imaging operation, and a step group 503 as post-processing for connecting data after the imaging operation is carried out, etc. Each step in each step group will be described.

(step 504)

Imaging blocks are indicated as the prearranging step for the imaging operation. In this step, a positioning sagital section image of low spatial resolution (an image obtained by viewing an object laid down on his/her back from the side) is imaged as a scanogram, for example. The gradient and/or the size at which each site of the object is disposed is detected by detecting means, and the image is displayed on the display 20. The operator inputs two or more imaging blocks onto the displayed scanogram or the like in accordance with the situation under which each site of the object (imaging target area) is disposed (the gradient and/or the size, or the like) while watching the display 20. The input of the imaging blocks is carried out by inputting an oblong (rectangular shape), a parallelogram or the like onto the display 20 by using the track ball, the mouse 23, the keyboard 24 or the like in Fig. 1. However, since the imaging is continuously carried out under the same scanning condition in the image block, it is expected to be better that an imaging block to be set is as large as possible so that a broader range is imaged under the same scanning condition in accordance with the arrangement condition of each site of the object (imaging target area).

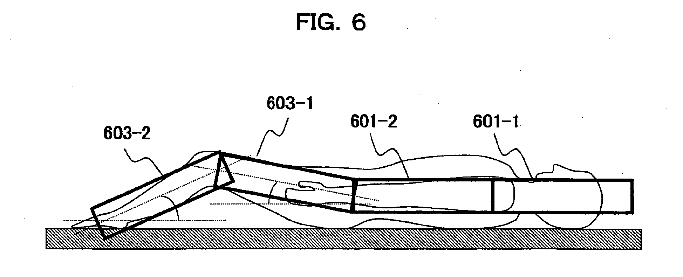

Fig. 6 shows an example of the set imaging block. According to Fig. 6, the upper half body of the object is in parallel direction with respect to the table plane. However, the lower half body is bent and thus is not parallel to the table plane. Therefore, in the setting of the imaging block of Fig. 6, an imaging block 601-1 and an imaging block 601-2 are stored in storage means (a memory contained in CPU 8 or the like) in parallel to the moving direction of the table for the upper half body, and they have cubic areas which are parallel to the moving direction of the table and contains the upper half body of the object. On the other hand, for the lower half body, an imaging block 601-3 and an imaging block 601-4 are stored in storage means not in parallel to the table plane, but so as to have a gradient in conformity with the orientation of the foot of the object, and they have rectangular parallelepiped areas which contain the feet of the object and are not parallel to the moving direction of the table plane.

(step 505)

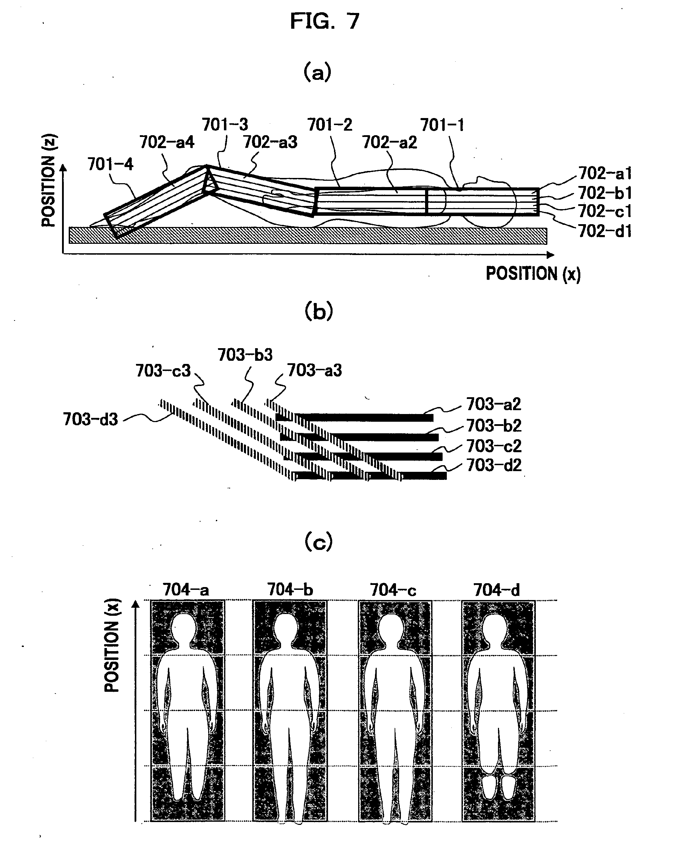

Next, the imaging condition of each imaging block is set. More specifically, Fig. 7 (a) shows a setting example when the multi-slice number is set to four. When the operator inputs the number of the imaging slice sections in each imaging block to the same number, four, by using the track ball or the mouse 23 and keyboard 24 or the like, a screen shown in Fig. 7(a) is displayed on the display 20. According to Fig. 7(a), with respect to an imaging block 701-1 and an imaging block 701-2, every four imaging slice sections in each imaging block are set in parallel to the moving direction of the table by the setting means. With respect to an imaging block 701-3 and an imaging block 701-4, the imaging slice sections are set not in parallel to the moving direction of the table, but so as to be inclined in conformity with the gradient of the imaging block 701-3 and the imaging block 701-4. The set information of the imaging slice section set in this step is temporarily stored in a magnetic disk 18. Here, four imaging slice sections out of the respective four slice sections set in the respective imaging blocks which are located at the uppermost position in the vertical direction are set as 702-a1 to 702-a4, four imaging slices which are located just below the uppermost imaging slice sections 702-a1 to 702-a4 in the vertical direction are set as 702-b1 to 702-b4, four imaging slice sections which are located just below the imaging slice sections 702-b1 to 702-b4 in the vertical direction are set as 702-c1 to 702-c4, and four imaging slice sections which are located at the lowest position in the vertical direction are set as 702-d1 to 702-d4. (In Fig. 7 (a), only 702-a1 to a4, 702-b1, 702-c1, 702-d1 are shown).

Fig. 9(a) shows a detailed diagram when imaging slice sections are indicated while the angle is varied every imaging block. In Fig. 9(a), the x axis corresponds to the moving direction of the table, the y axis corresponds to the direction along which the phase encode gradient magnetic field is applied, and the z axis corresponds to the vertical direction. According to Fig. 9(a), in a slab 901-1, the direction of the imaging slice section is parallel to the moving direction of the table, however, in a slab 901-2, the direction of the imaging slice section has a gradient θ with respect to the moving direction of the table. Here, the slab represents a set of plural multi-slices arranged in one imaging block.

In a case where the imaging slice sections are set as described above and the gradient echo pulse sequence is executed to perform imaging, when the reading gradient magnetic field output is represented by Gx(t) and the slice gradient magnetic field output is represented by Gz (t) in the operation of imaging the slab 901-1, the reading/slice gradient magnetic field output when the slab 901-2 is obtained is represented by the following equation 1.

[Equation 1]

$$\begin{cases} G_x{}'(t) = G_x(t) \cdot \cos\theta + G_z(t) \cdot \sin\theta \\ G_z{}'(t) = G_z(t) \cdot \cos\theta + G_x(t) \cdot \sin\theta \end{cases} \qquad (1)$$

(step 506)

The table is moved to the initial position to start the measurement. For example, when the imaging is started from the head portion of the object, the table is set so that the head portion of the object is disposed at the center of the imaging visual field.

(step 507)

The imaging is started while the table is slightly moved.

(step 508)

In the imaging operation which is carried out while the table is fed, it is judged whether the table is moved to the next imaging block set in step 504. If the table is moved to the next imaging block, the processing goes to step 509. If the table does not move to the next imaging bock, the processing goes to step 510.

(step 509)

When the table position is moved to the position of the next imaging block and the gradient of the arrangement of the imaging block is varied or the size of the imaging block is varied, the setting of the application of the gradient magnetic field pulse of the slice selection and the setting of the application of the reading gradient magnetic field pulse are changed on the basis of the setting of the imaging condition (stored information) for each block which is carried out in step 505 by the control means such as CPU 8 or the like so that the imaging of the next imaging block can be performed. For example, when the imaging block is moved from 701-2 to 701-3 in Fig. 7(a), the direction of the gradient magnetic field of the slice selection and the direction of the reading gradient magnetic field are inclined, and thus the setting is changed so that the oblique gradient magnetic field can be applied in conformity with each gradient. More specifically, when the imaging block is moved from the block 701-2 to the block 701-3, the switching is carried out so that on the x-z plane of Fig. 7(b) the imaging slice sections set in 703-a2 to 703-d2 are imaged, and then the imaging slice sections set in 703-a3 to 703-d3 are imaged.

Here, the imaging order of the respective imaging slice sections may be set like 703-d2 → 703-c2 → 703-b2 → 703-a2 → 703-a3 → 703-b3 → 703-c3 → 703-d3. Furthermore, the imaging slice sections represented by 703-a2 to 703-d2 and 703-a3 to 703-d3 may be partially overlapped with one another as shown in Fig. 7(b). When the imaging blocks are identical in orientation, but different in size, the setting on the degree of variation of the gradient magnetic field intensity of the slice selection is changed every time the gradient echo pulse sequence is executed.

(step 510)

The gradient magnetic field intensity of the slice selection, the phase encode amount, etc. are sequentially changed by the control means such as CPU 8 or the like, and the gradient echo pulse sequence is executed one by one. More specifically, in the imaging operation of this embodiment, the gradient echo pulse sequence is executed while the table is slightly moved and while the position of the imaging slice section for collecting the echo signal is changed one by one. The table is moved at a predetermined moving speed, and the gradient echo pulse sequence is successively carried out while the irradiation frequency of the RF pulse (201 in Fig. 2(a)) and the intensity of the gradient magnetic field of the slice selection (202 in Fig. 2 (a)) are successively changed, thereby successively collecting echo signals from the respective imaging slice sections. For example, when the number of multi-slices is equal to four, after the echo signals are successively collected from the four multi-slices (702-a to 702-d) in the imaging blocks, the table is returned to 702-a and successively fed while the slice selection is carried out till 702-d.

Here, the process of determining the position of the imaging slice section to be imaged while the table is fed will be described with reference to Fig. 8. In Fig. 8, (a) and (b) are examples in which the arrangement direction of the imaging blocks is parallel to the moving direction of the table like 701-1 or 701-2 in Fig. 7(a), and (c) and (d) of Fig. 8 are examples in which the arrangement direction of the imaging blocks is not parallel to the moving direction of the table, but has a gradient with respect to the moving direction of the table like 701-3 or 701-4 in Fig. 7(a). In Fig. 8, (a) and (c) represent the x-ky (x-PE) plane whose abscissa axis represents the position x of the moving direction of the table and whose ordinate axis represents the phase encode amount, and (b) and (d) represent the x-z plane whose abscissa axis represents the position x of the moving direction of the table and whose ordinate axis represents the slice position z of the vertical direction. Furthermore, Figs. 8(b) and (d) are cross-sectional views taken along A-A' in Figs. 8(a) and (c).

In the execution of the gradient echo pulse sequence described below, at the position of 801-1 in Fig. 8 (a), the imaging is successively carried out in the order of 801-1a, 801-1b, 801-1c and 801-1d in section of Fig. 8(b). Subsequently, the phase encode amount is incremented by one step, and the imaging position is shifted to the position of 801-2 on the x-ky plane of Fig. 8 (a) to successively image the imaging slice sections having the same heights in the z direction as 801-1a, 801-1b, 801-1c, 801-1d of Fig. 8(b). Thereafter, the phase encode amount is successively incremented by one step, and the imaging is carried out according to an arrow 802 in Fig. 8 (a) till 801-7. After the imaging till 801-7 is finished, the imaging position is returned to the same amount as the phase encode amount 801-1, and the imaging on the imaging slice sections at the position of 801-8 of the x-ky section of Fig. 8(a) is carried out. Thereafter, the phase encode amount is incremented by one step, and the imaging is carried out according to an arrow 803.

The same is applied to the cases of Figs. 8 (c) and (d) in which the imaging blocks are inclined with respect to the moving direction of the table. At the position of 804-1 of Fig. 8(c), the imaging is successively carried out in the order of 804-1a, 804-1b, 804-1c and 804-1d in section of Fig. 8(d). Subsequently, the phase encode amount is incremented by one step, and the imaging position is shifted to the position of 804-2 on the x-ky plane of Fig. 8(c) to successively image the imaging slice sections having the same heights in the z direction as 804-1a, 804-1b, 804-1c, 804-1d of Fig. 8(c). Thereafter, the phase encode amount is successively incremented by one step, and the imaging till 804-7 is carried out according to an arrow 805 in Fig. 8 (c) . When the imaging till 804-7 is finished, the imaging position is returned to the same amount as the phase encode amount 804-1, and the imaging slice sections at the position of 804-8 of the x-ky section of Fig. 8 (c) are imaged. Thereafter, the phase encode amount is incremented by one step, and the imaging is carried out according to an arrow 806.

(step 511)

It is judged whether the table reaches the final moving position and all necessary echo signals can be collected. If all the echo signals can be collected, the processing goes to step 512. If all the echo signals cannot be collected, the processing goes to step 508.

(step 512)

The echo signal data obtained in each block are read from the magnetic disk 18 to the memory in CPU 8 and the connecting processing is carried out by the combining means in CPU 8.

The connecting method is carried out in conformity with the method described with reference to Fig. 4(d). That is, the obtained echo signal data are subj ected to one-dimensional Fourier Transform in the x-direction (the table moving direction) and then connected to one another by the connecting means disposed in CPU 8 in the combining means along the arranged imaging blocks in Fig. 7(a). For example, with respect to this connection, the uppermost imaging slice sections, 702-a1 to 702-d1, of the imaging slice sections slice-arranged in the respective imaging blocks 4 are connected to one another and set as 704-a so that the spatial positional arrangement is matched among the data to be connected, the imaging slice sections 702-a1 to 702-d1 below the imaging slice sections 702-a1 to 702-d1 in the vertical direction are connected to one another and set as 704-b, the imaging slice sections 703-a1 to 703-d1 below the imaging slice sections 702-a1 to 702-d1 in the vertical direction are connected to one another and set as 704-c, and the imaging slice sections 704-a1 to 704-d1 below the imaging slice sections 703-a1 to 703-d1 in the vertical direction are connected to one another and set as 704-d, thereby generating the hybrid data described with reference to Fig. 4(d).

(step 513)

The data (704-a to 704-d) connected in step 512 are subjected to Fourier Transform in the phase encode direction (the ky direction in Fig. 4(d)) to create the whole images. This calculation is carried out in CPU 8, and the obtained result is shown in Fig. 7(c). The whole images 705-a to 705-d are shown in connection with the hybrid data 704-a to 704-d. According to Fig. 7 (c), in this embodiment, a portion at which the imaging slice section being oblique is provided, and thus the images are shown as a continuous image without being discontinued at the knee portion and the end portion of the foot.

(step 514)

The whole images created in the whole image creating step 513 are displayed on the display 20, for example. The whole image data are temporarily stored in the magnetic disk 18.

As described above, in the embodiment 1, the imaging section can be optimally set corresponding to the arrangement condition (the gradient, etc.) of the imaging target site.

Embodiment 2

An embodiment 2 of the present invention will be described with reference to Fig. 9. This embodiment relates to a case where the set imaging block is parallel to the moving direction of the table and a case where the set imaging block is not parallel to the moving direction of the table and has a gradient with respect to the moving direction of the table. In this embodiment, hybrid data obtained from imaging blocks each of which is not parallel and has a gradient are subjected to data interpolation processing to calculate values on a grid arranged in parallel to the moving direction of the table. Thereafter, the hybrid data after the data interpolation processing between the respective imaging blocks are correctly connected to one another, and subjected to Fourier Transform in the phase encode direction to obtain a final image. This embodiment is different only the step 511 of the flowchart shown in Fig. 5 of the embodiment 1, and the step 511 is replaced by step 511a. Accordingly, only the portion concerned will be described.

(step 511a)

In this step, the echo signal data obtained in the respective slabs are subjected to Fourier Transform in the direction of the application of the reading gradient magnetic field to create hybrid data. This aspect is shown in Fig. 9 (b) and Fig. 9(c). In Fig. 9(b), 902-1 at the left side is obtained by directly arranging the echo signal data measured in the slab 901-1 of Fig. 9(a). 903-1 at the right side is obtained by subjecting 902-1 to Fourier Transform in the direction of the application of the reading gradient magnetic field, making the position thereof on the x-axis proper and arranging the result as hybrid data. Furthermore, 902-2 at the left side of Fig. 9(c) is obtained by directly arranging the echo signal data measured in the slab 901-2 of Fig. 9 (a), and 903-2 at the right side is obtained by subjecting 902-2 to Fourier Transform in the direction of the application of the reading gradient magnetic field, making the position thereof on the x-axis proper and arranging the result as hybrid data. According to the hybrid data arranged at the right side in Figs. 9 (b) and (c), it is found that each hybrid data is displaced in conformity with the movement of the table little by little.

Next, when the hybrid data obtained in Figs. 9(b) and (c) is added with the position information in the z-axis direction and arranged in a virtual three-dimensional space, the result as shown in Fig. 9(d) is obtained, for example. In Fig. 9(d), the left side corresponds to the slab 901-2, and the right side corresponds to the slab 901-1. When the slab 901-1 is parallel to the moving direction of the table as shown at the right side, the hybrid data are put on the grid disposed in parallel to the moving direction of the table, and thus no special conversion is required. However, when the slab 901-2 is inclined with respect to the moving direction of the table as shown at the left side, it is required to execute the interpolation processing and calculate the values on the grid disposed in parallel to the moving direction of the table.

Fig. 9 (e) is an enlarged view of a part on a dashed line 904 of Fig. 9 (d), and solid lines 905-1 and 905-2 represent imaging slice sections in the slab 901-2, 906-1 to 906-7 represent data on the imaging slice sections, and 907-1 to 907-5 represent a part of the grid disposed in parallel to the moving direction of the table.

In the example shown in Fig. 9(e), the positions of the data on the imaging slice section and the positions of the grid points disposed in parallel to the moving direction of the table are not coincident with one another in many cases. Therefore, the points on the grid are interpolatively determined. The interpolation processing is executed for each grid point from 907-1 till 907-5. In this embodiment, the description will be typically made on 907-3.

When the value on the grid point 907-3 is determined, a predetermined range is settled as a square 908, for example, and the interpolation processing is executed with hybrid data on the imaging slice sections within the range by interpolating means contained in CPU 8. In the example of Fig. 9(e), the following equation 2 is calculated by using 906-2, 906-3, 906-5.

[Equation 2]

$$P(m) = \frac{\displaystyle\sum_{n=1}^{N(m)} W(r(m,n)) \cdot D(m,n)}{\displaystyle\sum_{n=1}^{N(m)} W(r(m,n))} \qquad (2)$$

In the equation (2), P(m) represents an m-th ($1 \leq m \leq M$: M represents the total number of grid points) grid point 907-3, N(m) represent the number of data within an effective range around the m-th grid point, D (m,n) represents the value of an n-th measured data within an effective range around an m-th noted grid point, r(m,n) represents the distance between the position of the noted grid point m and the position of the measured data n, and W(r(m,n)) represents the weighting function corresponding to the distance. A Sinc function represented by the equation 3 may be considered as an example of the weighting function W (r) (in this case, $\alpha$ represents any distance) .

[Equation 3]

$$W(r) = \begin{cases} \sin c(\pi|r|) = \dfrac{\sin(\pi|r|)}{\pi|r|} & (|r| \leq \alpha) \\ 0 & (|r| > \alpha) \end{cases} \qquad (3)$$

909 at the left side of Fig. 9(f) is obtained by restoring the hybrid data in the slab 901-2 to data on the grid points by the interpolation and representing the data on the x-ky plane as described above.

909 in Fig. 9 (f) can be connected to the hybrid data 903-1 obtained in the slab 901-1, and if they are connected to each other, the right side of Fig. 9(f) is obtained.

When the hybrid data connected as shown at the right side of Fig. 9 (f) are further subjected to Fourier Transform in the ky direction, the final whole image is obtained.

As described above, according to the second embodiment, even when an imaging slice section is indicated while varying the gradient every imaging block, the spatial information of the whole image can be correctly reconstructed by connecting the data which are obtained through the interpolation processing by using the position information.

Embodiment 3

An embodiment 3 will be described with reference to Fig. 10. This embodiment is different from the flowchart of Fig. 5 of the embodiment 1 in that only the step 504, the step 505 and the step 511 are replaced by step 504b, step 505b and step 511b, and only this portion will be described. This embodiment relates to an imaging method which is performed by varying the size of the imaging block, the direction of the imaging slice section disposed in each imaging block and the direction of the application of the reading gradient magnetic field among the imaging blocks. The steps 504b, 505b and the step 511b of this embodiment will be described in turn.

(step 504b)

In the setting of the imaging block of this step, the size of the imaging block is varied every imaging block. Fig. 10 (a) shows a setting example of this step. According to Fig. 10(a), 1001-1 having a large thickness is set in the vertical direction (z direction) to pick up images of the breast portion and the abdominal part of the object, and 1001-2 having a small thickness is set in the vertical direction (z direction) to pick up images of the leg portion of the object.

(step 505b)

In the setting of the imaging slice section of this embodiment, the setting direction of the imaging slice section is varied every imaging block, and further the direction of the reading gradient magnetic field to be applied for imaging is also varied every imaging slice section.
Fig. 10 (b) shows a setting example of the directions of the imaging slice section, the reading gradient magnetic field and the phase encode gradient magnetic field. According to Fig. 10(b), in an imaging block 1001-1, the imaging slice section is set in the vertical direction to the x-axis direction, the direction of the reading gradient magnetic field is set to the z-axis direction, and the direction of the phase encode gradient magnetic field is set to the y-axis direction and indicated by 1002-1. In an imaging block 1001-2, the imaging slice section is set vertically to the z-axis direction, the direction of the reading gradient magnetic field is set to the x-axis direction, and the direction of the phase encode gradient magnetic field is set to the y-axis direction and indicated by 1002-2.

(step 511b)

In the step 511b of this embodiment, the obtained echo signal data are subjected to one-dimensional Fourier Transform in the direction of the application of the reading gradient magnetic field. When the obtained data are arranged on the virtual three-dimensional hybrid space, they become as shown in Fig. 10 (c) and Fig. 10 (d). However, Fig. 10(c) is a cross-sectional view obtained by cutting the hybrid data disposed on the virtual three-dimensional hybrid space by a section parallel to the x-ky plane, and Fig. 10 (d) is a cross-sectional view obtained by cutting the hybrid data by a section parallel to the x-z plane. Furthermore, Fig. 10 (c) shows data at any position in the z-axis direction, and Fig. 10 (d) shows data at any position in the ky-axis direction. In the figures, dashed lines and solid lines correspond to hybrid data generate from one echo signal, and the arrows in the figures represent the time order of collecting the echo signals for generating the respective hybrid data.

The table moves to the plus direction of the x-axis during measurement little by little, and the hybrid data obtained by each echo signal are moved to the minus direction of the x-axis little by little. Furthermore, in the imaging block 1001-1, the imaging slice section is on the y-z plane, and thus the arrangement of the hybrid data on the x-ky plane of Fig. 10(c) becomes a dot arrangement. However, the arrangement of the hybrid data on the x-z plane of Fig. 10 (d) is on a line. On the other hand, with respect to the imaging block 1001-2, since the imaging slice section is on the x-y plane, the arrangement of the hybrid data on the x-ky plane of Fig. 10(c) is linear. However, the arrangement on the x-z plane of Fig. 10(d) is on a line.

(step 512a)

The data interpolation processing is executed on the data arranged in the virtual three-dimensional hybrid space shown in Figs. 10(c) and (d), and the values of the hybrid data on a predetermined grid are calculated. For example, the values at the points on the grid which are indicated by dashed lines in Figs. 10 (c) and (d) are calculated and set as hybrid interpolated data. Furthermore, the hybrid interpolation data are subjected to Fourier Transform in the ky direction to obtain the final whole image. However, the hybrid interpolation data in Fig. 10 (d) contain no data corresponding to the positions in the z-axis direction of 1003-2 and 1003-3, and thus these portions may be embedded with data having zero value.

As described above, according to the second embodiment, even when the imaging is carried out while varying the reading direction among the imaging blocks, by matching the position information and connecting the data, the reconstruction can be performed so that the spatial information of the whole image is corrected. This embodiment is an effective method when it is better to perform the imaging by a different imaging method in order to obtain a more excellent image in accordance with the imaging site of the object.

Embodiment 4

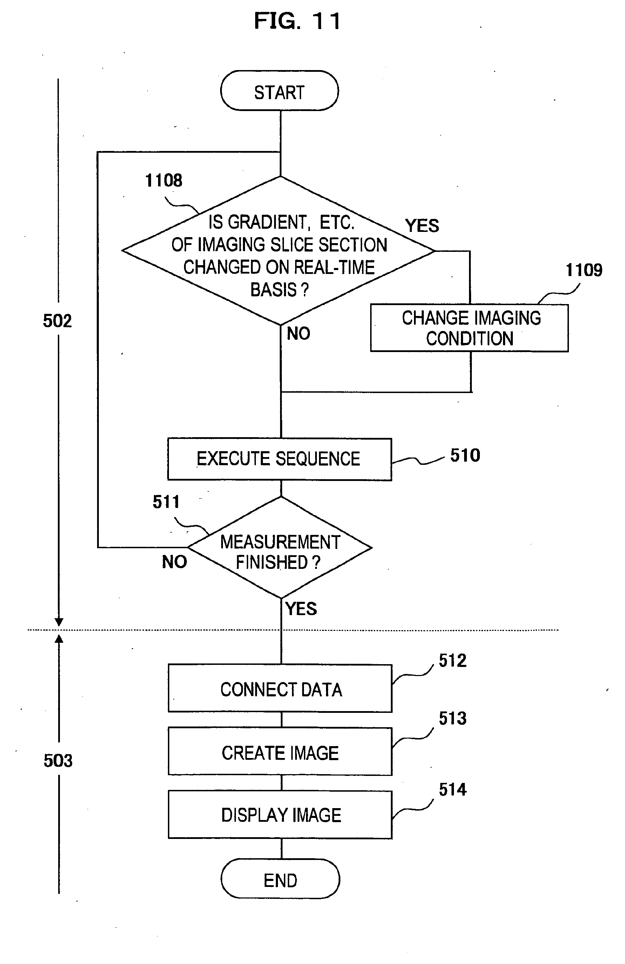

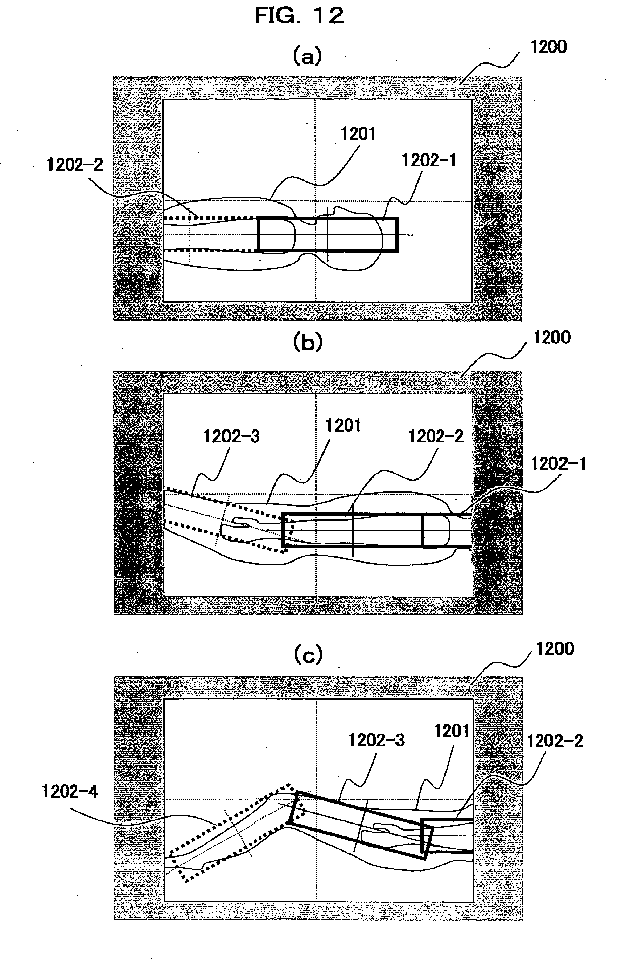

An embodiment 4 of the present invention will be described with reference to the flowchart of Fig. 11 and Fig. 12. However, in this embodiment, the imaging slice section is changed on a real-time basis in the act of continuously imaging the object while moving the table. Next, this embodiment will be described with reference to the flowchart of Fig. 11. As compared with Fig. 5 of the embodiment 1, Fig. 11 has no step group 501 and has step 1108 and step 1109 in place of the step group 501, and thus only the steps 1108 and 1109 will be described.

(step 1108)

In this step, the gradient or the like of the imaging slice section is changed on a real-time basis by using a graphic user interface or the like during the imaging operation. If it is changed, the processing goes to step 1109, and if not so, the processing goes to step 510.

(step 1109)

The change of the imaging condition such as the gradient of the imaging slice section or the like is input on a real-time basis. This aspect will be described with reference to Fig. 12. Fig. 12 shows a positioning image of the object (for example, a view obtained when an object laid down on his/her back is viewed from the side thereof) on the display 20. In Fig. 12, 1201 represents the object, and 1202-1 and 1202-2 represent boxes representing imaging slice sections. In this embodiment, the box representing the imaging slice section is displayed while a present imaging position, a past imaging-executed position and an imaging scheduled position are discriminated from one another.

For example, in the case of Fig. 12 (a), the solid box indicated by 1202-1 indicates that's the imaging slice section in the box concerned is under imaging, and the box of the dashed line indicated by 1202-2 indicates that the imaging slice section in the box will be next imaged. When the table is moved with time lapse, the imaging slice section is moved to the abdominal direction of the object and the abdominal part side of the positioning image of the object is displayed in connection with the movement of the imaging slice section. Fig. 12(b) shows the positioning image after a predetermined time elapses from the time of Fig. 12(a), and the box 1202-2 is indicated by the solid line, and thus becomes a box under imaging. At the timing of Fig. 12(b), the box 1202-3 to be next imaged is input so as to be inclined in conformity with the inclination of the legs of the object. For this input, the track ball, the mouse 23, the keyboard 24 or the like in Fig. 1 is used.

Fig. 12 (c) shows a result when the imaging further progresses from the timing of Fig. 2(b). According to Fig. 12 (c), 1202-2 becomes a box under imaging and thus is indicated by a solid line. At the timing of Fig. 12 (c), the box 1202-4 to be next imaged is input so as to be inclined in conformity with the inclination of the legs of the object. In step 510, the sequence is executed on the basis of the change of the imaging condition input in step 1109 or the like. The above operation is repeated until the imaging is finished, whereby the imaging slice section can be freely renewed during imaging.

In this embodiment, when the box to be next imaged is set so as to be inclined in step 1108 during imaging, the setting for application of an oblique gradient magnetic field or the like is carried out on the basis of the input information in step 1109 so that the next box can be imaged, and the gradient echo pulse sequence can be executed in conformity with the inclination in step 510. Therefore, the imaging slice section can be changed on a real-time basis in the act of continuously imaging the object while moving the table.

The present invention is not limited to the above embodiments, and various modifications may be made without departing from the subject matter of the present invention. For example, in this embodiment, the imaging is carried out on the basis of the gradient echo pulse sequence, however, other sequences may be applied. Furthermore, the data connection processing is carried out after all the measurements are finished. However, the connection processing may be carried out immediately at the time when the necessary data are collected even during measurement.

Furthermore, the frequency of changing the section during imaging is set to twice or three times. However, the changing frequency is not limited to these values. Furthermore, in this embodiment, the multi-slice based measurement is used as the method of achieving three-dimensional data. However, the three-dimensional measurement may be carried out by using the encode gradient magnetic field in the slice direction.

In the above embodiment, the gradient may be varied with respect to the moving direction of the table among imaging blocks or boxes. However, in order to well connect the hybrid data or the like on the basis of the data obtained in the respective imaging blocks or boxes, the areas of the echo signals collected in the respective imaging blocks or boxes may be partially overlapped with one another, whereby disconnection thereof in the connection step can be prevented, and it is preferable.

Furthermore, in the above-described embodiment, the table is continuously movable as in the case of the Non-patent Document 1. However, it is needless to say that the present invention may be applied to such a case that the imaging is carried out while the table is moved stepwise as in the case of the Patent Document 1.

Furthermore, the weighting function used for the data interpolation is not limited to the Sinc function, and it is needless to say that other functions such as Kaiser Bessel function, etc. may be used. It is unnecessary to pick up scanograms or the like to detect the gradient or the size at which each site of the obj ect is disposed, and the object may be imaged from the side by using a camera or the like.

Furthermore, it is unnecessary to input plural imaging blocks corresponding to the arrangement condition of each site of the object, and a straight line, a broken line, a curved line may be used.

Still furthermore, the moving speed of the table is not necessarily fixed, and it is needless to say that the moving speed may be increased or reduced during operation (for example, during the shift from 804-7 to 804-8 in Fig. 8(c) or the like) or stopped during operation in order to perform the imaging operation properly.

**Claims**

1. A magnetic resonance imaging apparatus that is equipped with static magnetic field generating means for generating static magnetic field in an imaging space, gradient magnetic field generating means for generating gradient magnetic field in the imaging space, radio frequency magnetic field generating means for generating radio frequency magnetic field so as to induce nuclear magnetic resonance to an object disposed in the imaging space, signal receiving means for detecting a nuclear magnetic resonance signal from the object, signal processing means for reconstructing an image by using the detected nuclear magnetic resonance signal, display means for displaying the image, a table for disposing the object in the imaging space while the object is put on the table, and table moving means for moving the table on which the object is put and in which the overall image of the object is obtained while moving the object in the imaging space continuously, or stepwise, with respect to each imaging site, thereby performing magnetic resonance imaging, **characterized by** further comprising:

   detecting means for detecting the gradient and size of each site of the object, the gradient and size of each site of the object that is detected by the detecting means being displayed on the display means;
   input means for inputting reference information for carrying out magnetic resonance imaging corresponding to the gradient and the size onto an image representing the gradient and size of each site of the object that is displayed on the display means;
   storage means for storing the input reference information;
   control means for controlling the imaging operation on the basis of the reference information stored in the storage means; and
   combining means for combining nuclear magnetic resonance signals obtained through the imaging operation executed under the control to generate the overall image.

2. The magnetic resonance imaging apparatus according to claim 1, wherein the detecting means detects by imaging a positioning image representing the whole of the object, the positioning image is displayed on the display means, the input means inputs the reference information onto the positioning image, and the control means controls occurrence of the gradient magnetic field and the radio frequency magnetic field by the gradient magnetic field generating means and the radio frequency magnetic field generating means so that imaging is carried out on the basis of the reference information, and controls detection of the nuclear magnetic resonance signal by the signal receiving means.

3. The magnetic resonance imaging apparatus according to claim 2, wherein the reference information is input as plural area information onto the positioning image.

4. The magnetic resonance imaging apparatus according to claim 3, wherein the area information is input and displayed as a rectangle representing plural rectangular parallelepiped areas containing each site of the object onto the display means.

5. The magnetic resonance imaging apparatus according to claim 4, wherein the rectangle is disposed along the gradient of each site.

6. The magnetic resonance imaging apparatus according to claim 4 or 5, wherein the input means inputs setting of plural imaging slice sections into the rectangular parallelepiped area.

7. The magnetic resonance imaging apparatus according to claim 6, wherein the plural imaging slice sections are set along the gradient.

8. The magnetic resonance imaging apparatus according to claim 7, wherein the number of the plural imaging slice sections is set to be equal among the respective rectangular parallelepiped areas, and the combining means is provided with connecting means for successively combining data obtained by processing the nuclear magnetic resonance signal occurring from each imaging slice section in each rectangular parallelepiped area, thereby forming the whole image.

9. The magnetic resonance imaging apparatus according to claim 8, wherein the data are hybrid data obtained by subjecting the nuclear magnetic resonance signal to one-dimensional Fourier Transform, and the connecting means connects the hybrid data among different rectangular parallelepiped areas in consideration of the spatial position information of the connection portion.

10. The magnetic resonance imaging apparatus according to claim 9, wherein the combining means is provided with interpolating means for conducting interpolation processing on the hybrid data based on the imaging slice section disposed so as to have a gradient with respect to the moving direction of the table, and calculating data on a grid disposed in parallel to the moving direction.

11. The magnetic resonance imaging apparatus according to any one of claims 3 to 9, wherein the plural area information contain size information that is different from the other areas.

12. The magnetic resonance imaging apparatus according to claim 6, wherein the plural area information contain area information having different directions of the imaging slice section or the applying directions of the reading gradient magnetic field.

13. A magnetic resonance imaging method in which a broad range or the whole body of an object is imaged while moving a table on which the object is laid down, **characterized by** comprising:

   (1) a step of inputting reference information in accordance with an arrangement situation of each site of the object;
   (2) a step of performing imaging by using the reference information; and
   (3) a step of synthesizing an overall image by using nuclear magnetic resonance signals obtained through the step (2) .

14. The magnetic resonance imaging method according to claim 13, further comprising (4) a step of picking up a positioning image representing the whole of the object before the step (1), wherein input of reference information in the step (2) is carried out on the positioning image.

15. The magnetic resonance imaging method according to claim 13 or 14, wherein the step (1) contains (5) a step of inputting area information representing plural areas containing respective sites of the object onto the positioning image, and (6) a step of inputting information for setting imaging slice sections in the plural area information.

16. The magnetic resonance imaging method according to any one of claims 13 to 15, wherein the step (2) contains (7) a step of judging whether the imaging slice section to be imaged to obtain a nuclear magnetic resonance signal next has been moved to the different area while moving the table, (8) a step of carrying out setting for applying proper gradient magnetic field or radio frequency magnetic field and detecting a nuclear magnetic resonance signal in accordance with the judgment of the step (7), and (9) a step of carrying out imaging in accordance with the setting carried out in the step (8).

17. The magnetic resonance imaging method according to any one of claims 13 to 16, wherein the step (3) comprises (10) a step of subjecting the nuclear magnetic resonance signal obtained in the step (2) to one-dimensional Fourier Transform, (11) a step of adding the data obtained through the one-dimensional Fourier Transform of the step (10) with spatial information thereof and arranging as hybrid data, and (12) a step of subjecting the hybrid data to Fourier Transform in the applying direction of phase encode gradient magnetic field to create a whole image.

18. The magnetic resonance imaging method according to any one of claims 15 to 17, wherein the area information and the imaging slice section are arranged along the gradient of each site.

19. The magnetic resonance imaging method according to any one of claims 15 to 18, wherein the number of the plural imaging slice sections is set to be equal among the respective areas, and the step (3) contains (13) a step of successively connecting data obtained by processing the nuclear magnetic resonance signal occurring from each imaging slice section in each area in order to generate the whole image.

20. The magnetic resonance imaging method according to claim 19, where the data are hybrid data obtained by subjecting the nuclear magnetic resonance signal to one-dimensional Fourier Transform, and the step (13) connects the hybrid data among different rectangular parallelepiped areas in consideration of spatial position information at connection portions.

21. The magnetic resonance imaging method according to claim 20, wherein the step (3) contains (14) a step of conducting interpolation processing on hybrid data based on imaging slice sections arranged having a gradient with respect to the moving direction of the table to obtained data on a grid disposed in parallel to the moving direction.

22. The magnetic resonance imaging method according to any one of claims 15 to 21, wherein the plural area information contain rectangles different in size.

23. The magnetic resonance imaging method according to any one of claims 13 to 17, wherein there are contained rectangles in which the direction along which the imaging slice section is set or the direction along which reading gradient magnetic field is applied is different among the plural rectangular parallelepiped areas.

24. The magnetic resonance imaging method according to any one of claims 22 and 23, wherein the rectangles are rectangular parallelepiped areas containing respective sites of the object.

# FIG. 1

EP 1 803 393 A1

# FIG. 2

## (a)

## (b)

# FIG. 3

## (a)

## (b)

## FIG. 4

(a)

(b)

(c)

(d)

# FIG. 5

START

**501**

INDICATE IMAGING BLOCK / **504**

SET IMALGING CONDITION / **505**

MOVE TABLE TO INITIAL POSITION / **506**

START IMAGING / **507**

**508**

MOVED TO NEXT IMAGING BLOCK ? — **YES** → CHANGE IMAGING CONDITION / **509**

**NO**

**502**

EXECUTE SEQUENCE / **510**

**511**

MEASUREMENT FINISHED ?  **NO**

**YES**

CONNECT DATA / **512**

CREATE IMAGE / **513**

**503**

DISPLAY IMAGE / **514**

END

## FIG. 6

# FIG. 7

## (a)

## (b)

## (c)

# FIG. 8

(a)

(b)

(c)

(d)

# FIG. 9

(a)

(b)

(c)

(d)

(e)

(f)

# FIG. 10

(a)

(b)

(c)

(d)

# FIG. 11

START

**1108**

IS GRADIENT, ETC. OF IMAGING SLICE SECTION CHANGED ON REAL-TIME BASIS ?

YES

**1109**

CHANGE IMAGING CONDITION

NO

**502**

EXECUTE SEQUENCE — **510**

**511**

MEASUREMENT FINISHED ?

NO

YES

**512**

CONNECT DATA

**513**

CREATE IMAGE

**514**

DISPLAY IMAGE

**503**

END

# FIG. 12

## (a)

1200

1201

1202-2

1202-1

## (b)

1200

1202-3

1201

1202-2

1202-1

## (c)

1200

1202-3

1201

1202-2

1202-4

## INTERNATIONAL SEARCH REPORT

| | |
|---|---|
| International application No. |
| PCT/JP2005/018781 |

A. CLASSIFICATION OF SUBJECT MATTER
**A61B5/055**(2006.01)

According to International Patent Classification (IPC) or to both national classification and IPC

B. FIELDS SEARCHED

Minimum documentation searched (classification system followed by classification symbols)
**A61B5/055**(2006.01)

Documentation searched other than minimum documentation to the extent that such documents are included in the fields searched
Jitsuyo Shinan Koho          1922-1996     Jitsuyo Shinan Toroku Koho    1996-2006
Kokai Jitsuyo Shinan Koho    1971-2006     Toroku Jitsuyo Shinan Koho    1994-2006

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | David G. Kruger et al., Continuously Moving Table Data Acquisitino Method for Long FOV Contrast-Enhanced MRA and Whole-Body MRI, Magnetci Resonance in Medicine, 2002, February, Vol.47, No.2, pages 224 to 231 | 1-12 |
| A | US 6311085 B1 (James F.M. Meaney), 30 October, 2001 (30.10.01), Column 8, lines 19 to 26 (Family: none) | 1-12 |
| A | JP 2002-95646 A (Toshiba Corp.), 02 April, 2002 (02.04.02), Column 13, lines 4 to 10; Fig. 5 & US 2002/0115929 A1 | 1-12 |

☒ Further documents are listed in the continuation of Box C.          ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 06 January, 2006 (06.01.06) | 17 January, 2006 (17.01.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**EP 1 803 393 A1**

**INTERNATIONAL SEARCH REPORT**

International application No.

PCT/JP2005/018781

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2003-135429 A  (GE Medical Systems Global Technology Co. LLC.), 13 May, 2003 (13.05.03), Column 11, lines 34 to 36; column 12, lines 5 to 8, 27 to 46 & US 2002/0140423 A1    & DE 10246406 A1 | 1-12 |
| A | JP 2003-290171 A  (Toshiba Iyo System Engineering Kabushiki Kaisha), 14 October, 2003 (14.10.03), Column 5, line 23 to column 6, line 10; column 7, lines 4 to 9; Fig. 5 & US 2003/0139660 A1    & CN 1432341 A | 1-12 |

Form PCT/ISA/210 (continuation of second sheet) (April 2005)

**EP 1 803 393 A1**

## INTERNATIONAL SEARCH REPORT

International application No.

PCT/JP2005/018781

**Box No. II   Observations where certain claims were found unsearchable (Continuation of item 2 of first sheet)**

This international search report has not been established in respect of certain claims under Article 17(2)(a) for the following reasons:

1. ☒ Claims Nos.: 13-24
because they relate to subject matter not required to be searched by this Authority, namely:
The inventions of claims 13-24 relate to a magnetic resonance imaging method including a step for inputting reference information in accordance with the arrangement condition of each portion of an examinee and performing imaging by using the reference information.  (Continued to extra sheet)

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III   Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**
the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

**INTERNATIONAL SEARCH REPORT**

International application No.

PCT/JP2005/018781

Continuation of Box No.II-1 of continuation of first sheet(2)

  Accordingly, the inventions fall in diagnostic methods practiced on the human body.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6311085 B

- JP 7023931 A

**Non-patent literature cited in the description**

- **KRUGER DG ; RIEDERER SJ ; GRIMM RC ; ROSSMAN PJ.** Continuously Moving Table Data Acquisition Method for Long FOV Contrast-Enhanced MRA and Whole-Body MRI. *Magnetic Resonance in Meddicine,* 2002, vol. 47 (2), 224-231